(19) Europäisches Patentamt
European Patent Office
Office européen des brevets

(11) Veröffentlichungsnummer: **0 175 225**
**A2**

# (12) EUROPÄISCHE PATENTANMELDUNG

(21) Anmeldenummer: **85111251.6**

(22) Anmeldetag: **06.09.85**

(51) Int. Cl.⁴: **C 07 D 251/70**, C 07 D 251/50, C 07 F 9/09, D 06 M 13/34

(30) Priorität: **15.09.84 DE 3433983**

(43) Veröffentlichungstag der Anmeldung: **26.03.86 Patentblatt 86/13**

(84) Benannte Vertragsstaaten: **CH DE FR GB LI SE**

(71) Anmelder: **HOECHST AKTIENGESELLSCHAFT, Postfach 80 03 20, D-6230 Frankfurt am Main 80 (DE)**

(72) Erfinder: **Springer, Hartmut, Dr., Am Erdbeerstein 27, D-6240 Königstein/Taunus (DE)**
Erfinder: **Russ, Werner Hubert, Dr., Berliner Strasse 10, D-6238 Hofheim am Taunus (DE)**

## (54) Faserreaktive Triazinverbindungen, Verfahren zu ihrer Herstellung und ihre Verwendung.

(57) Beschrieben sind Verbindungen der allgemeinen Formel (1)

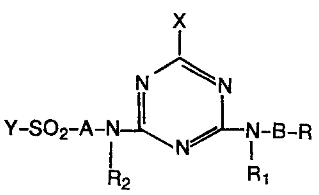

(1)

in welcher
A und B jeweils ein Alkylen von 2 bis 6 C-Atomen sind oder ein Phenylen- oder Naphthylenrest, die durch Methyl, Ethyl, Methoxy, Ethoxy, Chlor, Brom, Cyan, Alkanoylamino von 2 bis 5 C-Atomen, Nitro, Hydroxy, Carboxy und/oder Sulfo substituiert sein können,
Y β-Sulfatoethyl, β-Thiosulfatoethyl, β-Phosphatoethyl, β-Chlorethyl oder Vinyl bedeutet,
R für Wasserstoff oder eine Gruppe der allgemeinen Formel $-SO_2-Z$ steht, in welcher Z β-Sulfatoethyl, β-Thiosulfatoethyl, β-Phosphatoethyl, β-Chlorethyl oder Vinyl ist,
$R^1$ und $R^2$ jedes Wasserstoff oder Alkyl von 2 bis 6 C-Atomen sind, das durch Hydroxy, Sulfo, Carboxy oder Cyan oder eine Gruppe der Formel $-SO_2-Z$ substituiert sein kann,
X Chlor oder eine Gruppe der allgemeinen Formel

$-O-R^3$, $-S-R^3$, 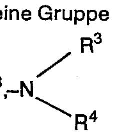 ist,

in welchen
$R^3$ Wasserstoff, ein gegebenenfalls durch Chlor, Methyl, Ethyl, Methoxy, Ethoxy, Carboxy und/oder Sulfo substituiertes Phenyl oder Alkyl von 2 bis 6 C-Atomen ist, das durch Hydroxy, Sulfo, Carboxy, Cyan oder eine Gruppe der Formel $-SO_2-Z$ substituiert sein kann, und
$R^4$ Wasserstoff oder Alkyl von 2 bis 6 C-Atomen ist, das durch Hydroxy, Sulfo, Carboxy oder Cyan oder eine Gruppe der Formel $-SO_2-Z$ substituiert sein kann, oder ein Phenyl oder Naphthyl bedeutet, die durch Halogen, Methyl, Ethyl, Methoxy, Ethoxy, Sulfo und Carboxy und/oder einer Gruppe der Formel $-SO_2-Z$ substituiert sein können, wobei die Verbindungen (1) mindestens eine der in den obigen Definitionen angegebenen wasserlöslichmachenden Gruppen und zwingend mindestens zwei faserreaktive Gruppen, die aus den Gruppen der Formeln $-SO_2-Y$, $-SO_2-Z$ und X gleich Chlor ausgewählt sind, enthalten,
jedoch nicht beschrieben sind Verbindungen der Formel (1), in welcher gleichzeitig X eine Aminogruppe $-NR^3R^4$ mit $R^4$ gleich einem durch eine Gruppe der Formel (2) substituierten Phenyl-

oder Naphthylrest, R eine Gruppe der Formel (2) und A und B jeweils einen unsubstituierten oder substituierten Phenylen- oder Naphthylenrest bedeuten.

Die Verbindungen (1) dienen zur Modifizierung und Verbesserung der physikalischen und färberischen Eigenschaften von Materialien aus natürlichen und/oder synthetischen Polyamidfasern und/oder Polyurethanfasern sowie als Hilfsmittel in Färbeprozessen solcher Fasermaterialien.

HOECHST AKTIENGESELLSCHAFT HOE 84/F 216 Dr.ST

# Faserreaktive Triazinverbindungen, Verfahren zu ihrer Herstellung und ihre Verwendung

Die Erfindung liegt auf dem technischen Gebiet von wasserlöslichen, polyfunktionell-faserreaktiven Triazinverbindungen, die faserveredelnde Eigenschaften besitzen.

Eine Triazinverbindung dieses Typs ist aus dem Beispiel 1 der US-PS 3 278 253 bekannt.

Mit der vorliegenden Erfindung wurden nunmehr andere polyfunktionell-faserreaktive Triazinverbindungen entsprechend einer allgemeinen Formel (1)

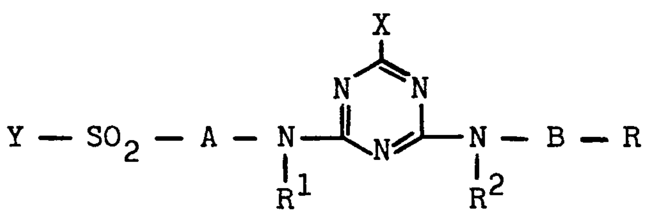

(1)

gefunden, in welcher bedeuten:

A ist eine geradkettige oder verzweigte Alkylengruppe von 2 bis 6 C-Atomen oder ist ein Phenylen- oder Naphthylenrest, die durch 1, 2 oder 3, bevorzugt 1 oder 2, Substituenten aus der Gruppe Methyl, Ethyl, Methoxy, Ethoxy, Chlor, Brom, Cyan, Alkanoylamino von 2 bis 5 C-Atomen, wie Acetylamino, Nitro, Hydroxy, Carboxy (entsprechend der allgemeinen Formel -COOM mit M der nachstehend angegebenen Bedeutung) und Sulfo (entsprechend der allgemeinen Formel $-SO_3M$ mit M der nachstehend angegebenen Bedeutung) substituiert sein können, wobei die Phenylenreste, sofern sie substituiert sind, bevorzugt 1 oder 2 Substituenten enthalten, die aus der Menge von 2 Methyl-, 2 Ethyl-, 2 Methoxy-, 2 Ethoxy-, 1 Cyan-, 1 Alkanoylamino-, 1 Nitro-, 1 Hydroxy-, 2 Carboxy- und

2 Sulfogruppen sowie 2 Chlor- und 2 Bromatomen ausgewählt sind, und wobei die Naphthylenreste bevorzugt durch 1 oder 2 Sulfogruppen substituiert sind;

B ist eine geradkettige oder verzweigte Alkylengruppe von 2 bis 6 C-Atomen oder ist ein Phenylen- oder Naphthylenrest, die durch 1, 2 oder 3, bevorzugt 1 oder 2, Substituenten aus der Gruppe Methyl, Ethyl, Methoxy, Ethoxy, Chlor, Brom, Cyan, Alkanoylamino von 2 bis 5 C-Atomen, wie Acetylamino, Nitro, Hydroxy, Carboxy und Sulfo substituiert sein können, wobei die Phenylenreste, sofern sie substituiert sind, bevorzugt 1 oder 2 Substituenten enthalten, die aus der Menge von 2 Methyl-, 2 Ethyl-, 2 Methoxy-, 2 Ethoxy-, 1 Cyan-, 1 Alkanoylamino-, 1 Nitro-, 1 Hydroxy-, 2 Carboxy- und 2 Sulfogruppen sowie 2 Chlor- und 2 Bromatomen ausgewählt sind, und wobei die Naphthylenreste bevorzugt durch 1 oder 2 Sulfogruppen sustituiert sind;

Y ist eine ß-Sulfatoethyl-Gruppe (entsprechend der allgemeinen Formel $-CH_2-CH_2-OSO_3M$ mit M der nachstehend angegebenen Bedeutung), eine ß-Thiosulfatoethyl-Gruppe (entsprechend der allgemeinen Formel $-CH_2-CH_2-S-SO_3M$ mit M der nachstehend angegebenen Bedeutung), eine ß-Phosphatoethyl-Gruppe (entsprechend der allgemeinen Formel $-CH_2-CH_2-OPO_3M_2$ mit M der nachstehend angegebenen Bedeutung), die ß-Chlorethyl-Gruppe oder die Vinylgruppe;

R ist ein Wasserstoffatom oder eine Gruppe der allgemeinen Formel (2)

$$-SO_2-Z \qquad (2)$$

in welcher

Z eine ß-Sulfatoethyl-, ß-Thiosulfatoethyl-, ß-Phosphatoethyl-, ß-Chlorethyl- oder Vinylgruppe ist;

$R^1$ ist ein Wasserstoffatom oder eine verzweigte oder geradkettige Alkylgruppe von 2 bis 6 C-Atomen, die durch eine Hydroxy-, Sulfo-, Carboxy- oder Cyangruppe oder eine Gruppe der obengenannten und definierten allgemeinen Formel (2) substituiert sein kann;

$R^2$ ist ein Wasserstoffatom oder eine verzweigte oder geradkettige Alkylgruppe von 2 bis 6 C-Atomen, die durch eine Hydroxy-, Sulfo-, Carboxy- oder Cyangruppe oder eine Gruppe der obengenannten und definierten allgemeinen Formel (2) substituiert sein kann;

X ist ein Chloratom oder eine Gruppe der allgemeinen Formel (3a), (3b) oder (3c)

$$-O-R^3 \,,\qquad -S-R^3 \,,$$

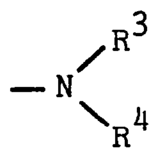

(3a) (3b) (3c)

in welchen

$R^3$ ein Wasserstoffatom oder ein gegebenenfalls durch Chlor, Methyl, Ethyl, Methoxy, Ethoxy, Carboxy und/oder Sulfo substituierter Phenylrest oder eine verzweigte oder geradkettige Alkylgruppe von 2 bis 6 C-Atomen, die durch eine Hydroxy-, Sulfo-, Carboxy- oder Cyangruppe oder eine Gruppe der obengenannten und definierten allgemeinen Formel (2) substituiert sein kann, ist und

$R^4$ ein Wasserstoffatom oder eine verzweigte oder geradkettige Alkylgruppe von 2 bis 6 C-Atomen, die durch eine Hydroxy-, Sulfo-, Carboxy- oder Cyangruppe oder eine Gruppe der obengenannten und definierten allgemeinen Formel (2) substituiert sein kann, darstellt oder einen Phenylrest oder einen Naphthylrest bedeutet, die beide durch 1, 2 oder 3, bevorzugt 1 oder 2, Substituenten substituiert sein können, die aus einer Gruppe von Halogenatomen, wie Chlor- und Bromatomen, Methyl-, Ethyl-, Methoxy-, Ethoxy-, Sulfo- und Carboxygruppen und einer Gruppe der obengenannten

und definierten allgemeinen Formel (2) ausgewählt sind;

jedoch ist X zwingend keine Aminogruppe der Formel (3c) mit $R^4$ gleich einem durch eine Gruppe der Formel (2) substituierten und durch andere der genannten Substituenten nicht oder zusätzlich substituierten Phenyl- oder Naphthylrest, falls R eine Gruppe der allgemeinen Formel (2) bedeutet und A und B gleichzeitig einen unsubstituierten oder substituierten Phenylen- oder Naphthylenrest bedeuten;

M steht für Wasserstoff, Lithium, Natrium, Kalium oder das Äquivalent des Calciums;

die Formelglieder A, B, X, Y, Z, R, $R^1$, $R^2$, $R^3$ und $R^4$ können nach Maßgabe der obengenannten Bedeutungen zueinander gleich oder voneinander verschieden sein;

die Verbindungen (1) enthalten mindestens eine der in den obigen Definitionen angegebenen wasserlöslich-machenden Gruppen, wie Sulfo-, Carboxy-, Sulfato- und Phosphatogruppe, bevorzugt mindestens eine Sulfogruppe, und enthalten zwingend mindestens 2 faserreaktive Gruppen, die aus den Gruppen der Formeln $-SO_2-Y$ , $-SO_2-Z$ und X gleich Chlor ausgewählt sind.

Die Substituenten in A und B, einschießlich der Gruppen $-SO_2-Y$ und R, können in beliebiger Stellung der aromatischen Kerne stehen.

Bevorzugt ist R eine Gruppe der allgemeinen Formel (2). Bevorzugt sind von den Gruppen Y und Z die Vinyl- und insbesondere die ß-Sulfatoethyl-Gruppen.

Bevorzugt sind weiterhin Verbindungen, in welchen $R^1$ und $R^2$ beide gleich sind und jedes ein Wasserstoffatom, eine Methylgruppe oder eine Ethylgruppe bedeutet, weiterhin insbesondere solche, in denen X ein Chloratom ist. Bevorzugt sind weiterhin solche Verbindungen der allgemeinen Formel (1), in denen die Formelglieder A und B,

zueinander gleich oder voneinander verschieden, jedes den Phenylenrest bedeutet, der durch 1 oder 2 Substituienten substituiert sein kann, die aus der Gruppe von 1 Chloratom, 2 Methoxygruppen, 1 Methylgruppe und 1 Sulfogruppe ausgewählt sind. Insbesondere bevorzugt sind Verbindungen der allgemeinen Formel (1), in welcher A und B beide den meta- oder para-Phenylenrest bedeuten, X für ein Chloratom, R für die Gruppe der Formel (2) und $R^1$ und $R^2$ beide für ein Wasserstoffatom stehen sowie Y und Z die oben angegebenen, insbesondere bevorzugten Bedeutungen haben.

Insbesondere sind bevorzugt Verbindungen entsprechend einer allgemeinen Formel (1a)

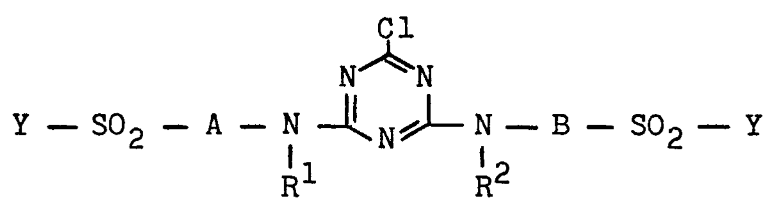
(1a)

in welchen A, B, $R^1$, $R^2$ und Y die obengenannten, insbesondere bevorzugten Bedeutungen haben.

Die vorliegende Erfindung betrifft weiterhin ein Verfahren zur Herstellung der Verbindungen der allgemeinen Formel (1), das dadurch gekennzeichnet ist, daß man Cyanurchlorid in äquimolaren oder etwa äquimolaren Mengen einer Aminoverbindung der allgemeinen Formel (4) und einer

0175225

Aminoverbindung der allgemeinen Formel (5) und gegebenenfalls einer Verbindung der allgemeinen Formel (6a), (6b) oder (6c)

$$Y - SO_2 - A - \underset{\displaystyle R^1}{\underset{|}{N}} - H \qquad (4)$$

$$H - \underset{\displaystyle R^2}{\underset{|}{N}} - B - R \qquad (5)$$

$$\underset{(6a)}{H\text{-}O\text{-}R^3} \; , \qquad \underset{(6b)}{H\text{-}S\text{-}R^3} \; , \qquad \underset{(6c)}{H\text{-}NR^3R^4} \; ,$$

in welchen A, B, R, $R^1$, $R^2$, Y, $R^3$ und $R^4$ die obengenannten Bedeutungen haben, umsetzt und hierbei die Ausgangsverbindungen so wählt, daß die Endverbindung der Formel (1) mindestens eine wasserlöslich machende Gruppe und mindestens zwei faserreaktive Gruppen aus den Gruppen der Formelglieder $-SO_2-Y$ , $-SO_2-Z$ und X gleich Chlor enthalten. Die Amine (4) und (5) werden in der Regel in einem bis 10%igen, vorzugsweise bis zu 5 %igen, Überschuß eingesetzt. Die Umsetzung der Verbindungen der Formeln (4), (5) und (6) erfolgt analog an und für sich bekannten und üblichen Verfahrensweisen der Umsetzung von aliphatischen und aromatischen Aminen, Alkoholen, Phenolen, Mercaptanen und Thiophenolen mit Cyanurchlorid, Dichlor-s-triazin-Verbindungen oder Monochlor-s-triazin-Verbindungen (s. bspw. Ullmanns Encyklopädie der technischen Chemie, 4. Auflage (1975), Band 9, S. 651). In der Regel erfolgt die Darstellung durch Umsetzung einer Di- bzw. Trichlor-s-triazin-Verbindung der allgemeinen Formel (7)

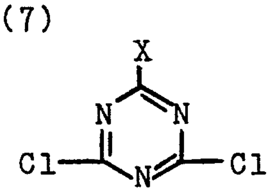

(7)

in welcher X die obengenannte Bedeutung besitzt, mit einem Amin der obengenannten allgemeinen Formel (4) und einem Amin der obengenannten allgemeinen Formel (5) in äquimolaren oder etwa äquimolaren Mengen, wobei die Umsetzung bevorzugt in einer Zweistufenreaktion durchgeführt wird und die Verbindung der Formel (7) zunächst mit der äquimolaren Menge eines Amins (4) oder (5) zur Verbindung der allgemeinen Formel (8)

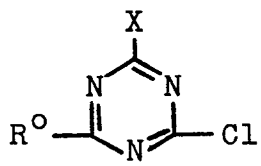

(8)

umsetzt, in welcher X die obengenannte Bedeutung besitzt und $R^o$ für einen Rest der allgemeinen Formel (9a) oder (9b)

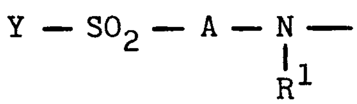

(9a)

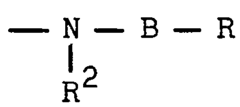

(9b)

mit A, B, $R^1$, $R^2$, R und Y der obengenannten Bedeutung steht, und anschließend, vorzugsweise ohne Zwischenisolierung, die Verbindung der Formel (8) mit dem Amin der allgemeinen Formel (5) bzw. (4) zur Verbindung (1) umsetzt. Sofern die Aminoverbindungen der allgemeinen Formeln (4) und (5) identisch sind, erübrigt sich die Umsetzung der Reaktionskomponenten unter getrennter Zugabe der beiden Amine.

Die Umsetzung des Cyanurchlorids oder einer Dichlor- oder Monochlor-s-triazin-Verbindung der allgemeinen Formel (8) kann in organischem oder wäßrig-organischem Medium erfolgen. Organische Lösemittel sind vorzugsweise Aceton und

Toluol. Vorzugsweise geschieht sie in wäßrigem Medium unter Zusatz säurebindender Mittel, wie Alkali- oder Erdalkalicarbonaten, Alkali- oder Erdalkalihydrogencarbonaten oder -hydroxiden oder Alkaliacetaten, wobei die Alkali- und Erdalkalimetalle vorzugsweise solche des Lithiums, Kaliums, Natriums und Calciums sind; säurebindende Mittel sind ebenso tertiäre Amine, wie beispielsweise Pyridin, Triethylamin oder Chinolin. Weiterhin kann der Zusatz von geringen Mengen eines handelsüblichen Hilfsmittels zweckmäßig sein.

Die Kondensationsreaktion des Cyanurchlorids mit einem Amin der allgemeinen Formel (4) oder (5) erfolgt in der Regel bei einer Temperatur zwischen -10°C und +40°C, vorzugsweise zwischen -10°C und +30°C, sowie bei einem pH-Wert zwischen 1,0 und 7,0, insbesondere zwischen 3,0 und 6,0.

Die erfindungsgemäße Umsetzung der Verbindungen der allgemeinen Formel (8) mit der entsprechenden Aminoverbindung der allgemeinen Formel (4) bzw. (5) erfolgt in der Regel bei einer Temperatur zwischen 0 und 70°C, insbesondere zwischen 10 und 50°C, und bei einem pH-Wert zwischen 2 und 9, insbesondere zwischen 3 und 8, wobei sowohl bei der Umsetzung des Cyanurchlorids als auch der Verbindungen der Formel (8) mit solchen Aminen der Formel (4) und/oder (5) darauf zu achten ist, daß der pH dann den Wert 7 nicht überschreitet, wenn das Amin eine ß-Thiosulfatoethylsulfonyl-, ß-Sulfatoethylsulfonyl- oder Vinylsulfonyl-Gruppe enthält.

Aminoverbindungen der allgemeinen Formel (4), die als Ausgangsverbindungen zur Herstellung der erfindungsgemäßen Verbindungen (1) dienen können, sind beispielsweise 4-(ß-Sulfatoethylsulfonyl)-N-methyl-anilin, 4-(ß-Sulfatoethylsulfonyl)-N-(ß-hydroxyethyl)-anilin, 4-(ß-Sulfatoethylsulfonyl)-N-(ß-cyanoethyl)-anilin, N,N-Bis-[ß-(ß'-Chlorethylsulfonyl)-ethyl]-amin, 4-(ß-Sulfatoethylsulfonyl)-phen-

0175225

ethylamin, 4-(ß-Sulfatoethylsulfonyl)-anilin, 3-(ß-Sulfatoethylsulfonyl)-anilin, 2-(ß-Sulfatoethylsulfonyl)-anilin, 3-(ß-Phosphatoethylsulfonyl)-anilin, 3-(ß-Sulfatoethylsulfonyl)-4-methyl-anilin, 3-(ß-Chlorethylsulfonyl)-anilin, 3-(ß-Sulfatoethylsulfonyl)-4-methoxy-anilin, 4-(ß-Sulfatoethylsulfonyl)-2-sulfo-anilin, 5-(ß-Sulfatoethylsulfonyl)-2-sulfo-anilin, 5-(ß-Sulfatoethylsulfonyl)-2,4-disulfo-anilin, 4-(ß-Sulfatoethylsulfonyl)-1-amino-naphthalin, 4-(ß-Sulfatoethylsulfonyl)-2,5-dimethoxy-anilin, 4-(ß-Sulfatoethylsulfonyl)-2-carboxy-anilin, 5-(ß-Sulfatoethylsulfonyl)-2-carboxy-anilin, 4-(ß-Sulfatoethylsulfonyl)-2-methoxy-anilin, 4-(ß-Sulfatoethylsulfonyl)-2-chlor-anilin, 5-(ß-Sulfatoethylsulfonyl)-2-methoxy-anilin, 8-(ß-Sulfatoethylsulfonyl)-2-amino-naphthalin, 8-(ß-Sulfatoethylsulfonyl)-6-sulfo-2-amino-naphthalin, 4-Vinylsulfonyl-2,5-dimethoxy-anilin, 4-(ß-Sulfatoethylsulfonyl)-2-methoxy-5-methyl-anilin, 4-(ß-Sulfatoethylsulfonyl)-2,5-diethoxy-anilin, 4-(ß-Sulfatoethylsulfonyl)-2-brom-anilin, 4-Vinylsulfonyl-2-brom-anilin, 5-Vinylsulfonyl-2,4-disulfo-anilin, 5-(ß-Phosphatoethylsulfonyl)-2,4-disulfo-anilin, 5-(ß-Chlorethylsulfonyl)-2,4-disulfo-anilin, 8-(ß-Phosphatoethylsulfonyl)-6-sulfo-2-amino-naphthalin, 8-Vinylsulfonyl-6-sulfo-2-amino-naphthalin, 4-(ß-Chlorethylsulfonyl)-2-methoxy-5-methyl-anilin, 4-(ß-Sulfatoethylsulfonyl)-2-aminophenol, 4-Vinylsulfonyl-anilin, 3-Vinylsulfonyl-anilin, 5-(ß-Sulfatoethylsulfonyl)-2-aminophenol, 5-Vinylsulfonyl-2-sulfo-anilin und ebenso deren entsprechenden ß-Thiosulfatoethylsulfonyl-, ß-Sulfatoethylsulfonyl-, Vinylsulfonyl-, ß-Phosphatoethylsulfonyl- und ß-Chlorethylsulfonyl-Abkömmlinge, sofern diese nicht bereits namentlich genannt wurden.

Aminoverbindungen der allgemeinen Formel (5), die als Ausgangsverbindungen zur Herstellung der erfindungsgemäßen Verbindungen (1) dienen können, sind beispielsweise 4-(ß-Sulfatoethylsulfonyl)-N-methyl-anilin, 4-(ß-Sulfatoethylsulfonyl)-N-(ß-hydroxyethyl)-anilin, 4-(ß-Sulfatoethylsulfonyl)-N-(ß-cyanoethyl)-anilin, N,N-Bis-[ß-(ß'-Chlorethylsulfonyl)-ethyl]-amin, 4-(ß-Sulfatoethylsulfonyl)-phenethylamin, 4-(ß-Sulfatoethylsulfonyl)-anilin, 3-(ß-Sulfatoethylsulfonyl)-anilin, 2-(ß-Sulfatoethylsulfonyl)-anilin, 3-(ß-Phosphatoethylsulfonyl)-anilin, 3-(ß-Sulfatoethylsulfonyl)-4-methyl-anilin, 3-(ß-Chlorethylsulfonyl)-anilin, 3-(ß-Sulfatoethylsulfonyl)-4-methoxy-anilin, 4-(ß-Sulfatoethylsulfonyl)-2-sulfo-anilin, 5-(ß-Sulfatoethylsulfonyl)-2-sulfo-anilin, 5-(ß-Sulfatoethylsulfonyl)-2,4-disulfo-anilin, 4-(ß-Sulfatoethylsulfonyl)-1-amino-naphthalin, 4-(ß-Sulfatoethylsulfonyl)-2,5-dimethoxy-anilin, 4-(ß-Sulfatoethylsulfonyl)-2-carboxy-anilin, 5-(ß-Sulfatoethylsulfonyl)-2-carboxy-anilin, 4-(ß-Sulfatoethylsulfonyl)-2-methoxy-anilin, 4-(ß-Sulfatoethylsulfonyl)-2-chlor-anilin, 5-(ß-Sulfatoethylsulfonyl)-2-methoxy-anilin, 8-(ß-Sulfatoethylsulfonyl)-2-amino-naphthalin, 8-(ß-Sulfatoethylsulfonyl)-6-sulfo-2-amino-naphthalin, 4-Vinylsulfonyl-2,5-dimethoxy-anilin, 4-(ß-Sulfatoethylsulfonyl)-2-methoxy-5-methyl-anilin, 4-(ß-Sulfatoethylsulfonyl)-2,5-diethoxy-anilin, 4-(ß-Sulfatoethylsulfonyl)-2-brom-anilin, 4-Vinylsulfonyl-2-brom-anilin, 5-Vinylsulfonyl-2,4-disulfo-anilin, 5-(ß-Phosphatoethylsulfonyl)-2,4-disulfo-anilin, 5-(ß-Chlorethylsulfonyl)-2,4-disulfo-anilin, 8-(ß-Phosphatoethylsulfonyl)-6-sulfo-2-amino-naphthalin, 8-Vinylsulfonyl-6-sulfo-2-amino-naphthalin, 4-(ß-Chlorethylsulfonyl)-2-methoxy-5-methyl-anilin, 4-(ß-Sulfatoethylsulfonyl)-2-aminophenol, 4-Vinylsulfonyl-anilin, 3-Vinylsulfonyl-anilin, 5-(ß-Sulfatoethylsulfonyl)-2-aminophenol, 5-Vinylsulfonyl-2-sulfo-anilin und ebenso deren entsprechenden ß-Thiosulfatoethylsulfonyl-, ß-Sulfatoethylsulfonyl-, Vinylsulfonyl-, ß-Phosphatoethylsulfonyl- und ß-Chlorethylsulfonyl-Abkömmlinge, sofern diese nicht bereits namentlich genannt wurden, weiterhin o-Toluidin, m-Toluidin, p-Toluidin, Anilin, 2,4-Dimethylanilin, 2,5-Dimethylanilin, 5-Methyl-2-methoxy-anilin, 2-Chlor-anilin, 3-Chlor-anilin, 4-Chlor-anilin, 2-Sulfo-anilin, 3-Sulfo-anilin, 4-Sulfo-anilin, 2,5-Disulfo-anilin, 3,5-Disulfo-anilin, 2,4-Disulfo-anilin, 4-Sulfo-anthranilsäure, 5-Sulfo-anthranilsäure, 2-Nitro-4-sulfo-anilin, 4-Nitro-2-sulfo-anilin, 3-Nitro-6-sulfo-anilin, 5-Nitro-4-sulfo-2-amino-anisol, 2-Methyl-4,5-disulfo-anilin, 2,5-Dichlor-4-sulfo-anilin, 4-Methyl-2-sulfo-anilin, 5-Chlor-4-methyl-2-sulfo-anilin, 4-Methoxy-2-sulfo-anilin, 4-Chlor-3-sulfo-anilin, 5-Acetylamino-2,4-disulfo-anilin, Anthranilsäure, 2-Amino-terephthalsäure, 3-Carboxy-anilin, 3-Amino-phthalsäure, 4-Amino-phthalsäure, 4-Carboxy-anilin, 5-Nitroanthranilsäure, 4-Nitro-3-carboxy-anilin, 5-Nitro-2-amino-terephthalsäure, 4-Sulfo-1-naphthylamin, 4,8-Disulfo-2-naphthylamin, 6-Nitro-4,8-disulfo-2-naphthylamin, 6,8-Disulfo-2-naphthylamin, 5,7-Disulfo-2-naphthylamin, 3-Acetylamino-6-sulfo-anilin, 4-Acetylamino-6-sulfo-anilin, 4-Sulfo-2-amino-anisol, 5-Sulfo-2-amino-anisol, 4-Hydroxy-3-carboxy-anilin, 1-Naphthylamin, 2-Methoxy-1-naphthylamin, 4-Sulfo-1-naphthylamin, 5-Sulfo-1-naphthylamin, 6-Sulfo-1-naphthylamin, 7-Sulfo-1-naphthylamin, 1-Sulfo-2-naphthylamin, 5-Sulfo-2-naphthylamin, 6-Sulfo-2-naphthylamin, 7-Sulfo-2-naphthylamin, 8-Sulfo-2-naphthylamin, 3,6-Disulfo-1-naphthylamin, 1,5-Disulfo-2-naphthylamin, 1,6-Disulfo-2-naphthylamin, 6-Acetylamino-4,8-disulfo-2-naphthylamin, 3,6,8-Trisulfo-2-naphthylamin, 4,6,8-Trisulfo-2-naphthylamin, 2,5,7-Trisulfo-1-naphthylamin.

Die Abscheidung und Isolierung der erfindungsgemäß hergestellten Verbindungen der allgemeinen Formel (1) aus den Syntheselösungen kann nach allgemein für faserreaktive wasserlösliche Verbindungen bekannten Methoden erfolgen, so beispielsweise durch Ausfällen aus dem Reaktionsmedium mittels Elektrolyten, wie mittels Natriumchlorid oder Kaliumchlorid, oder durch Eindampfen der Reaktionslösung,

bspw. durch Sprühtrocknung. Mutterlaugen, die sich aus den Reaktionsansätzen ergeben, oder Lösemittel, die zurückgewonnen werden, können vorteilhaft für weitere Folgeansätze im Kreislauf geführt werden. Auf diese Weise ist es möglich, die an und für sich in hoher Ausbeute erhältlichen wasserlöslichen Verbindungen der Formel (1) mit nur geringer oder gar ohne Abwasserbelastung durch anorganische Salze in effektiv hohen Ausbeuten zu isolieren.

Die neuen Verbindungen der allgemeinen Formel (1) dienen zur Modifizierung und Verbesserung der physikalischen und färberischen Eigenschaften von Materialien aus natürlichen und/oder synthetischen Polyamidfasern, wie Wolle, Polyamid-6, Polyamid-6,6 und Polyamid-11, und/oder Polyurethanfasern sowie als wertvolle Hilfsmittel in Färbeprozessen für solche Materialien. So verbessern sie beispielsweise die Egalität von Polyamidfärbungen, wenn man sie vor dem eigentlichen Färbeprozeß oder zusammen mit dem Farbstoff während des Färbeprozesses auf die natürliche und/oder synthetische Polyamidfaser einwirken läßt. In gleicher Weise gelingt es beispielsweise, die Naßreißfestigkeit von Wolle durch Behandlung mit den neuen Verbindungen der Formel (1) zu verbessern. Auch kann der Faserverlust, der üblicherweise beim Färben von Wolle bei Kochtemperatur (durch Hydrolyse) auftritt, mit Hilfe der neuen Verbindungen der Formel (1) vermindert werden, indem man das Wollgewebe oder die Wollflocke vor dem Färbeprozeß oder während desselben zusammen mit dem Farbstoff, beispielsweise einem Säurefarbstoff oder einem Reaktivfarbstoff, mit einer Verbindung der Formel (1), in einer Flotte gelöst bzw. in der erwähnten Färbeflotte selbst, behandelt. Die Einwirkung der Verbindungen der allgemeinen Formel (1) auf diese Fasermaterialien kann hierbei analog Verfahrensbedingungen und Verfahrensweisen, wie sie allgemein üblich und in bekannter Weise zum Färben dieser Fasermaterialien mittels faserreaktiver Farbstoffe angewendet werden, erfolgen (s. bspw. Seite 15, Zeilen 27 ff., bis Seite 16, Zeile 9,

sowie Seite 16, Zeile 23 ff., bis Seite 17, Zeile 23, der deutschen Offenlegungsschrift Nr. 3 132 917).

Die nachstehenden Beispiele dienen zur Erläuterung der Erfindung. Die Teile sind Gewichtsteile, die Prozentangaben stellen Gewichtsprozente dar, sofern nicht anders vermerkt. Gewichtsteile beziehen sich zu Volumenteilen wie Kilogramm zu Liter.

Beispiel 1

Es wird mittels etwa 100 Teilen Natriumcarbonat eine wäßrige Lösung mit einem pH-Wert von 4,5 von 562 Teilen 4-(ß-Sulfatoethylsulfonyl)-anilin in 2000 Teilen Wasser hergestellt; 0,5 Teile eines handelsüblichen Dispergierhilfsmittels werden zugegeben und anschließend bei einer Temperatur zwischen 15 und 20°C 184 Teile Cyanurchlorid in fester Form unter Rühren eingetragen. Unter Einhaltung des angegebenen pH-Wertes und des angegebenen Temperaturbereiches wird der Ansatz zwei Stunden weitergerührt, sodann auf 45 bis 50°C erwärmt und noch etwa vier Stunden bei einem pH-Wert von 4,5 (der mittels Natriumcarbonat gehalten wird) und bei dieser Temperatur zur Vervollständigung der Kondensationsreaktion weitergerührt.

Um das hergestellte erfindungsgemäße Produkt zu isolieren, wird der Ansatz auf 60°C erwärmt und mit soviel Wasser versetzt, bis dieses Produkt vollständig gelöst ist. Der Ansatz wird sodann filtriert und das Filtrat sprühgetrocknet oder eingedampft.

Man erhält etwa 825 Teile eines weiß-grauen Pulvers, das neben Elektrolytsalzen (wie Natriumchlorid) 86 % (entsprechend einer Ausbeute von 99,1 % d.Th.) der Verbindung der Formel

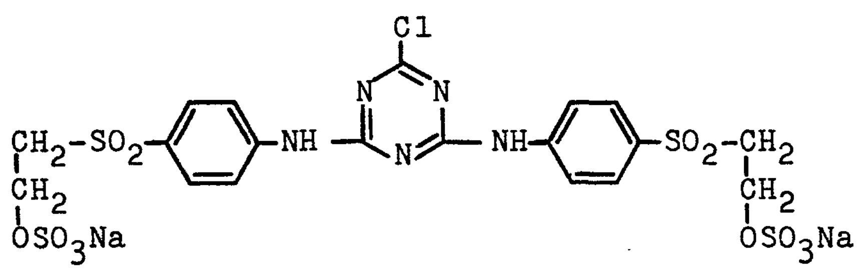

enthält. - Zur Charakterisierung kann der Schmelzpunkt des Divinyl-Derivates dienen. Hierzu wird die erfindungsgemäße Verbindung mit wäßriger Natronlauge bei Raumtemperatur in die entsprechende unlösliche Di-vinylsulfon-Verbindung übergeführt, die einen Zersetzungspunkt von 180°C besitzt.

Die erfindungsgemäße Verbindung verbessert bspw. die Naßreißfestigkeit von Wolle, wenn man Wolle in einem wäßrigen Bad mit der darin gelösten erfindungsgemäßen Verbindung behandelt (s. hierzu auch das spätere Anwendungsbeispiel).

Beispiel 2

a) Mittels etwa 54 Teilen Natriumcarbonat wird eine wäßrige Lösung mit einem pH-Wert von 4,5 von 281 Teilen 4-(ß-Sulfatoethylsulfonyl)-anilin in 5000 Teilen Wasser hergestellt, zu der 500 Teile Eis und 0,5 Teile eines handelsüblichen Dispergierhilfsmittels gegeben werden. Zur Herstellung des Monokondensationsproduktes aus diesem Amin und Cyanurchlorid werden sodann 184,5 Teile Cyanurchlorid in fester Form unter Rühren und unter Einhaltung eines pH-Wertes von 4,5 mittels Natriumcarbonat eingetragen; der Ansatz wird bei diesem pH-Wert und einer Temperatur zwischen 0 und 5°C noch 4 Stunden bis zur beendeten Kondensationsreaktion weitergerührt.

b) 173 Teile Sulfanilsäure werden in 500 Teilen Wasser mit 53 Teilen Natriumcarbonat gelöst; die Lösung wird zu der unter a) hergestellten Suspension des Monokondensationsproduktes gegeben. Die Reaktionsmischung wird auf 45 bis 50°C erwärmt und die Reaktion bei dieser Temperatur unter Rühren und unter Einhaltung eines pH-Wertes von 5,5 mittels etwa 55 Teilen Natriumcarbonat 2 Stunden weitergeführt.

Die Lösung des so hergestellten erfindungsgemäßen Produktes wird filtriert und das Filtrat sprühgetrocknet. Es werden 720 Teile eines hellgrauen Pulvers erhalten, das neben Elektrolytsalzen 83 % (entsprechend einer Ausbeute von 97,7 % d.Th.) der Verbindung der Formel

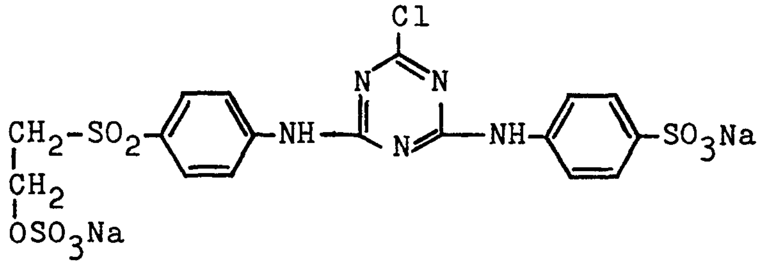

0175225

enthält. Die erfindungsgemäße Verbindung verbessert bspw. die Naßreißfestigkeit von Wolle, wenn diese in einem wäßrigen Bad, bspw. analog dem späteren Anwendungsbeispiel, mit der erfindungsgemäßen Verbindung behandelt wird.

Beispiel 3

Zur Herstellung einer erfindungsgemäßen Verbindung verfährt man gemäß der Verfahrensweise des Beispieles 2, setzt jedoch im Abschnitt b) in der zweiten Kondensationsreaktion anstelle der Sulfanilsäure 107 Teile N-Methylanilin ein.

Man erhält etwa 630 Teile eines weiß-grauen Pulvers, das die erfindungsgemäße Verbindung der Formel

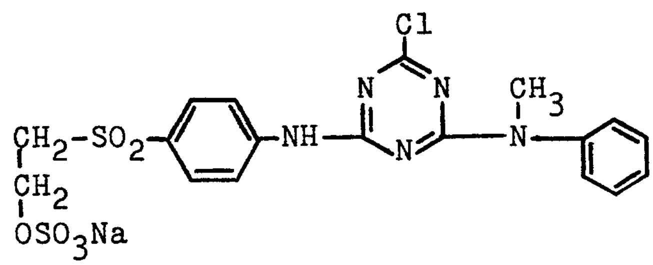

zu 80 % entsprechend einer Ausbeute von 96,6 % d.Th. enthält. Die erfindungsgemäße Verbindung verbessert bspw. die Naßreißfestigkeit von Wolle, wenn diese in einem wäßrigen Bad, bspw. analog dem späteren Anwendungsbeispiel, mit der erfindungsgemäßen Verbindung behandelt wird.

Beispiel 4

Zur Synthese des erfindungsgemäßen Vinylsulfonyl-Derivates der in Beispiel 2 beschriebenen Verbindung verfährt man zunächst gemäß der Verfahrensweise des Beispieles 2, stellt jedoch nach Filtration des Syntheseansatzes den pH des Filtrats bei einer Temperatur von 20°C mittels einer 30%igen wäßrigen Natronlauge auf einen Wert von 10 und hält ihn während 15 Minuten. Nach Neutralisation der Lösung

mittels konzentrierter wäßriger Salzsäure und Sprühtrocknung erhält man, in Form eines grauen, elektrolythaltigen Pulvers, die erfindungsgemäße Verbindung der Formel

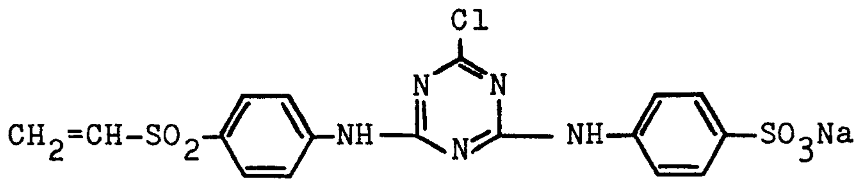

in einer Ausbeute von 87 % d.Th.. Die erfindungsgemäße Verbindung verbessert bspw. die Naßreißfestigkeit von Wolle, wenn diese in einem wäßrigen Bad, bspw. analog dem späteren Anwendungsbeispiel, mit der erfindungsgemäßen Verbindung behandelt wird.

Beispiel 5

Zur Herstellung einer erfindungsgemäßen Verbindung verfährt man gemäß der Verfahrensweise des Beispieles 2, setzt jedoch im Verfahrensschritt b) nach der zweiten Kondensationsreaktion noch zusätzlich 110 Teile Diethanolamin ein und führt die dritte Kondensationsreaktion während drei Stunden bei 80°C und einem pH-Wert von 6,0 durch. Nach Klärung des Syntheseansatzes und nach Sprühtrocknung des Filtrats erhält man ein leicht gelb gefärbtes elektrolythaltiges Pulver mit der erfindungsgemäßen Verbindung der Formel

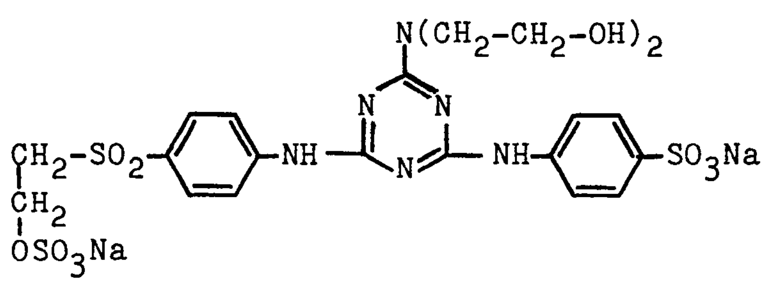

in einer Ausbeute von 85 % d.Th.. Die erfindungsgemäße Verbindung verbessert bspw. die Naßreißfestigkeit von Wolle, wenn diese in einem wäßrigen Bad, bspw. analog dem späteren

Anwendungsbeispiel, mit der erfindungsgemäßen Verbindung behandelt wird.

Beispiele 6 bis 22

In den nachfolgenden Tabellenbeispielen sind weitere erfindungsgemäße Verbindungen entsprechend der nachstehenden allgemeinen Formel (1b) beschrieben Bevorzugt liegen sie als Alkalimetallsalze, wie Natriumsalze, vor. Sie besitzen ebenfalls gute faserreaktive Eigenschaften und vermögen Wolle und synthetische Polyamidfasermaterialien in ihren physikalischen und färberischen Eigenschaften, wie beispielsweise für die erfindungsgemäße Verbindung des Beispieles 1 angegeben, zu modifizieren und zu verbessern. Die in den Tabellenbeispielen beschriebenen erfindungsgemäßen Verbindungen lassen sich in erfindungsgemäßer Verfahrensweise, beispielsweise analog den in den vorhergehenden Ausführungsbeispielen beschriebenen Methoden mit den aus den Tabellenbeispielen ersichtlichen Komponenten als Ausgangsverbindungen (der Aminoverbindung der allgemeinen Formel $A^*-NH_2$ , der Aminoverbindung der allgemeinen Formel $H_2N-B^*$ und dem Cyanurchlorid bzw. der Dichlor-s-triazin-Verbindung) herstellen.

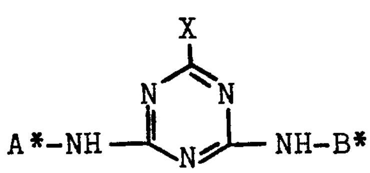

(1b)

| Bsp. | A* | B* | X |
|---|---|---|---|
| 6 | 2,6-Dibrom-4-(ß-sulfatoethylsulfonyl)-phenyl | 2,6-Dibrom-4-(ß-sulfatoethylsulfonyl)-phenyl | Chlor |
| 7 | 4-(ß-Sulfatoethylsulfonyl)-phenyl | ß-Sulfo-ethyl | Chlor |
| 8 | dito | ß-Hydroxy-ethyl | Chlor |
| 9 | dito | 3-Sulfo-phenyl | Chlor |
| 10 | dito | 3-Carboxy-phenyl | Chlor |
| 11 | dito | 6-(ß-Sulfatoethylsulfonyl)-2-sulfo-naphth-1-yl | Chlor |
| 12 | 3-(ß-Sulfatoethylsulfonyl)-phenyl | 4-(ß-Sulfatoethylsulfonyl)-phenyl | Chlor |
| 13 | dito | 3-(ß-Sulfatoethylsulfonyl)-phenyl | Chlor |
| 14 | 2-Methoxy-5-(ß-sulfatoethylsulfonyl)-phenyl | dito | Chlor |
| 15 | dito | 4-Sulfo-phenyl | Chlor |
| 16 | 2,5-Dimethoxy-4-(ß-sulfatoethylsulfonyl)-phenyl | dito | 2,5-Dimethoxy-4-(ß-sulfatoethylsulfonyl)-phenylamino |
| 17 | dito | 2,5-Dimethoxy-4-(ß-sulfatoethylsulfonyl)-phenyl | Chlor |
| 18 | 2-Methoxy-5-methyl-4-(ß-sulfatoethylsulfonyl)-phenyl | 2-Methoxy-5-methyl-4-(ß-sulfatoethylsulfonyl)-phenyl | Chlor |
| 19 | 2-Methoxy-5-methyl-4-(ß-sulfatoethylsulfonyl)-phenyl | 2-Methoxy-5-methyl-4-(ß-sulfatoethylsulfonyl)-phenyl | 2,5-Disulfo-phenylamino |
| 20 | 4-(ß-Chlorethylsulfonyl)-phenyl | 4-(ß-Chlorethylsulfonyl)-phenyl | dito |

| Bsp. | A* | B* | X |
|---|---|---|---|
| 21 | 4-(ß-Thiosulfato-ethylsulfonyl)-phenyl | 4-(ß-Thiosulfato-ethylsulfonyl)-phenyl | Chlor |
| 22 | 4-(ß-Phosphato-ethylsulfonyl)-phenyl | 4-(ß-Phosphato-ethylsulfonyl)-phenyl | Chlor |

Anwendungsbeispiel

100 Teile eines Wollgewebes werden in ein 40°C warmes wäßriges Bad eingebracht, das aus 3000 Teilen Wasser, 3 Teilen des Natriumsalzes der im Beispiel 1 beschriebenen erfindungsgemäßen Verbindung, 0,15 Teilen eines Anlagerungsproduktes von 12 Mol Ethylenoxid an 1 Mol Stearylamin, 2 Teilen Ammoniumacetat und 2 Teilen 60 %iger wäßriger Essigsäure besteht. Die Temperatur wird innerhalb von 30 Minuten auf Kochtemperatur erhöht und die Wolle anschließend 60 Minuten lang bei 100°C weiterbehandelt. Nach dem üblichen Auswaschen und Fertigstellen zeigt dieses Wollgewebe eine deutlich bessere Naßreißfestigkeit als unbehandelte Wolle.

PATENTANSPRÜCHE:

1. Verbindung entsprechend einer allgemeinen Formel (1)

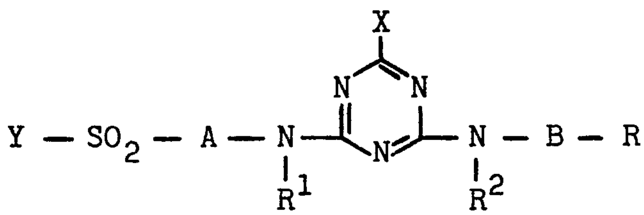 (1)

die mindestens eine der in den nachstehenden Definitionen angegebenen wasserlöslich-machenden Gruppen enthält und zwingend mindestens 2 faserreaktive Gruppen, die aus den Gruppen der Formeln $-SO_2-Y$ , $-SO_2-Z$ und X gleich Chlor ausgewählt sind, besitzt, wobei in Formel (1) bedeuten:

A ist eine geradkettige oder verzweigte Alkylengruppe von 2 bis 6 C-Atomen oder ist ein Phenylen- oder Naphthylenrest, die durch 1, 2 oder 3, bevorzugt 1 oder 2, Substituenten aus der Gruppe Methyl, Ethyl, Methoxy, Ethoxy, Chlor, Brom, Cyan, Alkanoylamino von 2 bis 5 C-Atomen, Nitro, Hydroxy, Carboxy und Sulfo substituiert sein können;

B ist eine geradkettige oder verzweigte Alkylengruppe von 2 bis 6 C-Atomen oder ist ein Phenylen- oder Naphthylenrest, die durch 1, 2 oder 3, bevorzugt 1 oder 2, Substituenten aus der Gruppe Methyl, Ethyl, Methoxy, Ethoxy, Chlor, Brom, Cyan, Alkanoylamino von 2 bis 5 C-Atomen, Nitro, Hydroxy, Carboxy und Sulfo substituiert sein können;

Y ist eine ß-Sulfatoethyl-, eine ß-Thiosulfato-ethyl-, eine ß-Phosphatoethyl-, die ß-Chlorethyl- oder die Vinylgruppe;

R ist ein Wasserstoffatom oder eine Gruppe der allgemeinen Formel (2)

$$-SO_2-Z \qquad (2)$$

in welcher

Z eine ß-Sulfatoethyl-, ß-Thiosulfatoethyl-, ß-Phosphatoethyl-, ß-Chlorethyl- oder Vinylgruppe ist;

$R^1$ ist ein Wasserstoffatom oder eine verzweigte oder geradkettige Alkylgruppe von 2 bis 6 C-Atomen, die durch eine Hydroxy-, Sulfo-, Carboxy- oder Cyangruppe oder eine Gruppe der obengenannten und definierten allgemeinen Formel (2) substituiert sein kann;

$R^2$ ist ein Wasserstoffatom oder eine verzweigte oder geradkettige Alkylgruppe von 2 bis 6 C-Atomen, die durch eine Hydroxy-, Sulfo-, Carboxy- oder Cyangruppe oder eine Gruppe der obengenannten und definierten allgemeinen Formel (2) substituiert sein kann;

X ist ein Chloratom oder eine Gruppe der allgemeinen Formel (3a), (3b) oder (3c)

$-O-R^3$ , $-S-R^3$ , 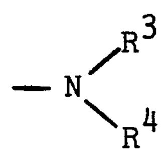

(3a) (3b) (3c)

in welchen

$R^3$ ein Wasserstoffatom oder ein gegebenenfalls durch Chlor, Methyl, Ethyl, Methoxy, Ethoxy, Carboxy und/oder Sulfo substituierter Phenylrest oder eine verzweigte oder geradkettige Alkylgruppe von 2 bis 6 C-Atomen, die durch eine Hydroxy-, Sulfo-, Carboxy- oder Cyangruppe oder eine Gruppe der obengenannten und definierten allgemeinen Formel (2) substituiert sein kann, ist und

$R^4$ ein Wasserstoffatom oder eine verzweigte oder geradkettige Alkylgruppe von 2 bis 6 C-Atomen, die durch eine Hydroxy-, Sulfo-, Carboxy- oder Cyangruppe oder eine Gruppe der obengenannten und definierten allgemeinen Formel (2) substituiert sein kann, darstellt oder einen Phenylrest oder einen Naphthylrest bedeutet, die beide durch

1, 2 oder 3 Substituenten substituiert sein können, die aus einer Gruppe von Halogenatomen, Methyl-, Ethyl-, Methoxy-, Ethoxy-, Sulfo- und Carboxygruppen und einer Gruppe der obengenannten und definierten allgemeinen Formel (2) ausgewählt sind;

ausgenommen jedoch solch eine Verbindung der Formel (1), in welcher gleichzeitig X eine Aminogruppe der Formel (3c) mit $R^4$ gleich einem durch eine Gruppe der Formel (2) substituierten und durch andere der genannten Substituenten unsubstituierten oder substituierten Phenyl- oder Naphthylrest, R eine Gruppe der allgemeinen Formel (2) und A und B jeweils einen unsubstituierten oder substituierten Phenylen- oder Naphthylenrest bedeuten.

2. Verbindung nach Anspruch 1, dadurch gekennzeichnet, daß $R^1$ und $R^2$ beide gleich sind und jedes ein Wasserstoffatom, eine Methylgruppe oder eine Ethylgruppe bedeuten.

3. Verbindung nach Anspruch 1 oder 2, dadurch gekennzeichnet, daß A und B, zueinander gleich oder voneinander verschieden, jedes einen Phenylenrest bedeutet, der durch 1 oder 2 Substituenten substituiert sein kann, die aus der Gruppe von 1 Chloratom, 2 Methoxygruppen, 1 Methylgruppe und 1 Sulfogruppe ausgewählt sind.

4. Verbindung nach Anspruch 1 oder 2, dadurch gekennzeichnet, daß A und B beide den meta- oder para-Phenylenrest bedeuten.

5. Verbindung nach Anspruch 1, dadurch gekennzeichnet, daß X für ein Chloratom steht.

6. Verbindung nach einem der Ansprüche 1 bis 4, dadurch gekennzeichnet, daß R für eine Gruppe der in Anspruch 1 genannten und definierten allgemeinen Formel (2) steht.

7. Verbindung nach einem der Ansprüche 1 bis 6, dadurch gekennzeichnet, daß Y und/oder Z für eine ß-Sulfatoethyl-Gruppe steht.

8. Verbindung nach Anspruch 1 entsprechend einer allgemeinen Formel (1a)

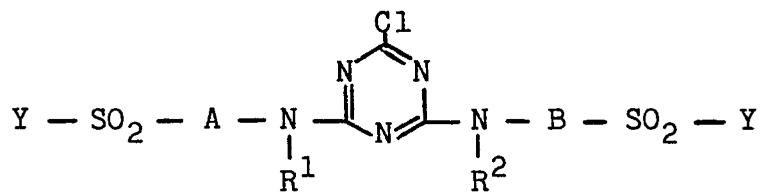 (1a)

in welcher A, B, $R^1$, $R^2$ und Y die in Anspruch 1 genannten Bedeutungen haben.

9. Verbindung nach Anspruch 8, in welcher $R^1$ und $R^2$, beide zueinander gleich oder voneinander verschieden, jedes ein Wasserstoffatom, eine Methylgruppe oder eine Ethylgruppe bedeutet, A und B, zueinander gleich oder voneinander verschieden, jedes einen meta- oder para-Phenylenrest bedeutet, der durch 1 oder 2 Substituenten substituiert sein kann, die aus der Gruppe von 1 Chloratom, 2 Methoxygruppen, 1 Methylgruppe und 1 Sulfogruppe ausgewählt sind, und beide Y eine ß-Sulfatoethyl-Gruppe bedeuten.

10. Verbindung nach Anspruch 9, dadurch gekennzeichnet, daß $R^1$ und $R^2$ jedes die gleiche Bedeutung besitzt und A und B jedes den gleichen unsubstituierten oder substituierten Phenylrest bedeutet.

11. Verbindung nach Anspruch 1, dadurch gekennzeichnet, daß Y und Z beide eine ß-Sulfatoethyl-Gruppe bedeuten.

12. Verfahren zur Herstellung einer in Anspruch 1 genannten und definierten Verbindung, dadurch gekennzeichnet, daß man in äquimolarer oder etwa äquimolarer Menge Cyanurchlorid mit einer Aminoverbindung der allgemeinen Formel (4) und einer Aminoverbindung der allgemeinen Formel (5) und gegebenenfalls einer Verbindung der allgemeinen Formel (6a), (6b) oder (6c)

$$Y - SO_2 - A - \underset{\underset{R^1}{|}}{N} - H \qquad (4)$$

$$H - \underset{\underset{R^2}{|}}{N} - B - R \qquad (5)$$

$$\underset{(6a)}{H - O - R^3}\ , \qquad \underset{(6b)}{H - S - R^3}\ , \qquad \underset{(6c)}{H - NR^3R^4}\ ,$$

in welchen A, B, R, $R^1$, $R^2$, Y, $R^3$ und $R^4$ in Anspruch 1 genannten Bedeutungen haben, umsetzt und hierbei die Ausgangsverbindungen so wählt, daß die Endverbindung der Formel (1) mindestens eine wasserlöslich machende Gruppe und mindestens zwei faserreaktive Gruppen aus den Gruppen der Formelglieder $-SO_2-Y$ , $-SO_2-Z$ und X gleich Chlor enthält.

13. Verfahren nach Anspruch 12, dadurch gekennzeichnet, daß man eine Verbindung der allgemeinen Formel (7)

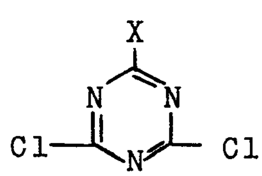

(7)

in welcher X die in Anspruch 1 genannte Bedeutung besitzt, mit einem Amin der in Anspruch 12 genannten und definierten allgemeinen Formel (4) oder einem Amin der in Anspruch 12 genannten und definierten allgemeinen Formel (5) in äquimolaren oder etwa äquimolaren Mengen zur Verbindung der allgemeinen Formel (8)

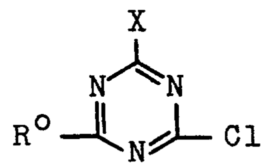

(8)

umsetzt, in welcher X die in Anspruch 1 genannte Bedeutung besitzt und $R^o$ für einen Rest der allgemeinen Formel (9a) oder (9b)

$$Y - SO_2 - A - \underset{R^1}{\underset{|}{N}} - \qquad (9a)$$

$$- \underset{R^2}{\underset{|}{N}} - B - R \qquad (9b)$$

mit A, B, $R^1$, $R^2$, R und Y der in Anspruch 1 genannten Bedeutung steht, und anschließend, vorzugsweise ohne Zwischenisolierung, die Verbindung der Formel (8) mit dem Amin der allgemeinen Formel (5) bzw. (4) zur Verbindung (1) umsetzt.

14. Verfahren nach Anspruch 12 zur Herstellung einer Verbindung von Anspruch 1, in welcher A, B, X, $R^1$, $R^2$ und Y die in Anspruch 1 genannten Bedeutungen haben und R für die in Anspruch 1 genannte Gruppe der allgemeinen Formel (2) steht und worin $R^1$ mit $R^2$ A mit B und R mit $-SO_2-Y$ jeweils gleich sind, dadurch gekennzeichnet, daß man eine Verbindung der allgemeinen Formel (7)

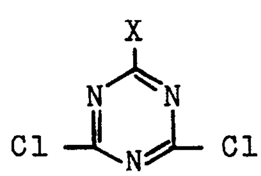

(7)

in welcher X die in Anspruch 1 genannte Bedeutung besitzt, mit der zweifach molaren Menge oder etwa zweifach molaren Menge eines Amins der allgemeinen Formel (4) umsetzt.

15. Verwendung einer Verbindung von Anspruch 1 zur Modifizierung und Verbesserung der physikalischen und färberischen Eigenschaften von Materialien aus natürlichen und/oder synthetischen Polyamidfasern und/oder Polyurethanfasern sowie als Hilfsmittel in Färbeprozessen solcher Fasermaterialien.